# EUROPÄISCHE PATENTSCHRIFT

(11) **EP 3 269 834 B1**
(45) Veröffentlichungstag und Bekanntmachung des Hinweises auf die Patenterteilung: **27.11.2019**
(21) Anmeldenummer: 16179717.0
(22) Anmeldetag: 15.07.2016
(51) Int. Cl.: C22C 5/02, A61B 18/14, A61B 18/00

(54) **ABLATIONSELEKTRODE AUF GOLDBASIS UND HERSTELLVERFAHREN**
GOLD-BASED ABLATION ELECTRODE AND PRODUCTION METHOD
ÉLECTRODE D'ABLATION A BASE D'OR ET PROCEDE DE FABRICATION

(43) Veröffentlichungstag der Anmeldung: 17.01.2018
(73) Patentinhaber: Heraeus Deutschland GmbH & Co. KG, 63450 Hanau (DE)
(72) Erfinder: Leitold, Christiane, 61200 Wölfersheim (DE); Keller, Thorsten, 64297 Darmstadt (DE); Buckow, Lydia, 63526 Erlensee (DE); Hammermeister, Christian, 63486 Bruchköbel (DE); Krämer, Winfried, 63619 Bad Orb (DE)
(74) Vertreter: Heraeus IP

(56) Entgegenhaltungen:
- EP-A2- 1 960 053
- US-A1- 2013 214 301

## Beschreibung

Die Erfindung bezieht sich auf eine Ablationselektrode für die Hochfrequenzablation in der Medizintechnik, wobei die Ablationselektrode eine Goldlegierung umfasst. Ferner betrifft die Erfindung eine Katheter-Spitze zur Verwendung mit einer Katheter-Vorrichtung, umfassend eine Ablationselektrode; sowie eine Katheter-Vorrichtung für die Hochfrequenzablation, umfassend eine Katheter-Spitze; sowie die Verwendung einer Ablationselektrode, einer Katheter-Spitze oder einer Katheter-Vorrichtung für die Hochfrequenzablation; sowie ein Verfahren zur Herstellung einer Ablationselektrode.

Bei Katheter-Ablationen wird Gewebe, das falsche elektrische Impulse sendet gezielt ausgeschaltet oder es werden Narben in Gewebebereichen verursacht, die die Weiterleitung der falschen Impulse unterbrechen. Bei der Hochfrequenzablation wird ein Katheter in das Gewebe eingebracht und eine lokal begrenzte Hitzezerstörung durch die Wärmeentwicklung eines angelegten Hochfrequenzstroms erzeugt. Die Hitzezerstörung geht von einer Ablationselektrode aus, die sich im Bereich der Spitze des distalen Endes einer Katheter-Vorrichtung befindet. Die Wärmeerzeugung in der Elektrode erfolgt in der Regel durch Hochfrequenz-Wellen mit Frequenzen von größer als 1 kHz.

Um eine ausreichende Wärmeabfuhr während der Behandlung des Patienten zu gewährleisten, müssen Katheter-Bauteile oft mit hohem konstruktivem Aufwand für eine ausreichende Wärmeabfuhr, beispielsweise durch Kühlkanäle, optimiert werden. Das US Patent 5,348,554 beschreibt eine Ablationselektrode, die eine interne Kühlung zur besseren Wärmeabfuhr vorsieht, weil durch das Bauteil selbst keine ausreichende Wärmeabfuhr gewährleistet ist.

Als Elektroden-Werkstoffe kommen allgemein in der Medizintechnik verwendete Platin-Iridium-Legierungen, vereinzelt auch Palladium-Legierungen, zum Einsatz. Für ungekühlte Katheter-Vorrichtungen oder Katheter-Vorrichtungen mit passiver Kühlung wären Legierungen mit höherer Wärmeleitfähigkeit als die der vorgenannten Legierungen wünschenswert.

US 6,099,524 beschreibt Mapping- und Ablationskatheter-Systeme mit Elektroden auf Basis von Gold, Goldlegierungen, Platin, Titan, Wolfram, Edelstahl und Cobalt-basierten biokompatiblen Materialien. Es wird eine Gold-Nickel-Legierung angegeben, beispielsweise eine Gold-Nickel-Legierung mit 88 Gew.-% Gold und 12 Gew.-% Nickel. Die genannte

Legierung soll eine höhere Wärmeleitfähigkeit als Platin-Legierungen aufweisen und findet daher als wärmeableitendes Material im Bereich der Katheter-Spitze eines Ablationskatheters Eine Ablationselektrode nach der Präambel von Anspruch 1 ist aus EP1960053 bekannt.

Legierungen wie AuNi12 oder andere Gold-Nickel-Legierungen erreichen ihre Festigkeit nur über den Prozess der Mischkristallverfestigung. Dadurch verfügen diese Legierungen über Wärmeleitfähigkeiten, die die von reinem Platin von 74 W/m*K nicht erreichen oder nur minimal überschreiten. Andere wirksame Verfestigungsmechanismen wie Ausscheidungshärtung sind für das AuNi Legierungssystem nicht bekannt. Dadurch ist es auch nicht möglich auf Basis von AuNi Legierungssystemen hohe mechanische Festigkeiten zu erzielen.

Reines Gold verfügt über eine spezifische Wärmeleitfähigkeit von 320 W/m*K, ist aufgrund seiner niedrigen Härte jedoch nur schwierig mit Hilfe von zerspanenden Verfahren wie Drehen, Fräsen und Schleifen bearbeitbar. Abtragende oder erodierende Verfahren wie Laserbearbeitung erfordern regelmäßig eine Haltevorrichtung, was bezüglich weicher Materialien wie Gold wiederum mit Problemen bei der Befestigung an derartigen Haltevorrichtungen verbunden ist. Dadurch ist es nicht möglich, Ablationselektroden in geringen Wandstärken auf der Basis von reinem Gold mit Hilfe dieser und ähnlicher Verfahren ökonomisch sinnvoll in großen Stückzahlen herzustellen.

Materialien mit einer hoher Wärmeleitfähigkeit wie der von Gold sind jedoch für die Anwendung in Ablations-Vorrichtungen insbesondere mit Elektroden von geringer Wandstärke wünschenswert, da aufgrund der hohen Wärmeleitfähigkeit eine effektive Wärmeabfuhr auch ohne aktive Kühlung möglich ist. Weiterhin sind Ablationselektroden mit geringer Wandstärke im Zuge fortschreitender Miniaturisierung in der Medizintechnik wünschenswert.

Allgemein ist es eine Aufgabe der vorliegenden Erfindung, die vorgenannten Nachteile des Standes der Technik wenigstens teilweise zu überwinden. Die Aufgabe der Erfindung ist insbesondere darin zu sehen eine Ablationselektrode bereitzustellen, die sowohl eine hohe spezifische Wärmeleitfähigkeit aufweist als auch ein Material beinhaltet, welches mit Hilfe zerspanender oder erodierender Verfahren in geringen Wandstärken herstellbar und bearbeitbar ist.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ablationselektrode für die Hochfrequenzablation in der Medizintechnik, wobei die Ablationselektrode eine Goldlegierung umfasst. Erfindungsgemäß ist vorgesehen, dass die Goldlegierung ausscheidungsgehärtet ist, wenigstens 80 Gew.-% Gold aufweist und 1 Gew.-% bis 9 Gew.-% Kobalt aufweist.

Reines Gold verfügt über eine hohe spezifische elektrische Leitfähigkeit und eine hohe spezifische Wärmeleitfähigkeit, ist für die Fertigung von Ablationselektroden mit geringen Wandstärken jedoch zu weich. Auf reinen Mischkristallen basierende Goldlegierungen verfügen oft über eine zu geringe Festigkeit. Zwar steigt mit dem Anteil des jeweiligen Legierungselements die Festigkeit; jedoch nimmt die elektrische und thermische Leitfähigkeit in der Regel in gleichem Maße ab. Die erfindungsgemäße ausscheidungsgehärtete Legierung verfügt über Ausscheidungen, die eine effektivere Verfestigung bewirken als reine Mischkristallbildungen. Gleichzeitig wurde überraschenderweise gefunden, dass die erfindungsgemäße Ablationselektrode auch über eine hohe elektrische und thermische Leitfähigkeit verfügt. Es wird vermutet dass die ausscheidungsgehärtete Goldlegierung einen geringen Gehalt an Legierungselementen, insbesondere Kobalt, in der Matrix aufweist. Kobalt reichert sich somit in den Ausscheidungen an. Der hohe Anteil Gold in der Matrix wirkt sich vermutlich positiv hinsichtlich der vorgenannten Eigenschaften aus.

In einer bevorzugten Ausführungsform der Ablationselektrode weist die Goldlegierung 1 Gew.-% bis 9 Gew.-% Kobalt und mindestens 90 Gew.-% Gold auf. Insbesondere weist die Goldlegierung 91 Gew. % bis 99 Gew. % Gold, bevorzugt 92 Gew.-% bis 98 Gew.-% Gold, besonders bevorzugt 94 Gew.-% bis 96 Gew.-% Gold und weiter bevorzugt 95 Gew.% Gold auf. In einer speziell bevorzugten Ausführungsform besteht die Goldlegierung aus 95 Gew.-% Gold und 5 Gew.-% Kobalt. Weitere nicht zu vermeidende Verunreinigungen in geringen Mengen, insbesondere von weiteren Metallen oder Elementen wie N, O oder S, können Bestandteil der Legierung sein.

Eine hohe Zahl und/oder Menge von weiteren Legierungselementen beeinträchtigt grundsätzlich die elektrische und thermische Leitfähigkeit der Goldlegierung. Daher sind Goldlegierungen mit binärer Zusammensetzung, insbesondere Goldlegierungen die ausschließlich Gold und Kobalt beinhalten, bevorzugte Ausführungsformen.

In einer bevorzugten Ausführungsform der Ablationselektrode weist die Goldlegierung 2 Gew.-% bis 8 Gew.-% Kobalt auf, besonders bevorzugt weist die Goldlegierung 4 Gew.-% bis 6 Gew.-% Kobalt auf und weiter bevorzugt weist die Goldlegierung 5 Gew.-% Kobalt auf.

Im Bereich zwischen 2 und 8 Gew.-% Kobalt weist das genannte Legierungssystem einen besonders hohen Ausscheidungsanteil Kobaltphase im Material auf, wodurch eine besonders effektive Verfestigung erreicht wird. Im Bereich zwischen 4 Gew.-% bis 6 Gew.-% Kobalt ist der Ausscheidungsanteil so hoch, dass eine besonders effektive Verfestigung kombiniert mit einer hohen elektrischen Leitfähigkeit und Wärmeleitfähigkeit erzielt wird. Für eine Goldlegierung mit einem Kobalt-Anteil von 5 Gew.-% ergibt sich durch die zu vernachlässigende Löslichkeit innerhalb der Gold- und Kobaltphase insbesondere im Temperatur-Bereich von 400 °C bis 500 °C ein Ausscheidungsanteil der Kobaltphase von annähernd 5 Gew.-% im Material. Ein Anteil von 5 Gew.-% Kobalt in der Goldlegierung ist daher nahezu als Idealwert zu sehen und dadurch besonders bevorzugt. Besonders bevorzugt besteht die Goldlegierung aus 2 bis 8 Gew.-% Kobalt, besonders bevorzugt aus 4 Gew.-% bis 6 Gew.-% Kobalt, und weiter bevorzugt aus 5 Gew.-% Kobalt und der restliche Anteil der Goldlegierung ist jeweils Gold.

In einer bevorzugten Ausführungsform der Ablationselektrode weist die Goldlegierung mindestens ein weiteres Legierungselement ausgewählt aus der Gruppe Platin, Iridium und Palladium auf. Besonders bevorzugt beträgt die Gesamtmenge der weiteren Legierungselemente weniger als 10 Gew.-%, weiter bevorzugt weniger als 5 Gew.-% und speziell bevorzugt weniger als 1 Gew.-%.

Die vorgenannten Platinmetalle zeichnen sich insbesondere durch eine gute Biokompatibilität, Reaktionsträgheit und damit verbundene Korrosionsbeständigkeit sowie gute elektrische und thermische Leitfähigkeit aus.

In einer bevorzugten Ausführungsform der Ablationselektrode weist die Goldlegierung eine spezifische Wärmeleitfähigkeit von wenigstens 100 W/m*K auf, bevorzugt von wenigstens 130 W/m*K auf, besonders bevorzugt von wenigstens 150 W/m*K auf. Dabei weist die Goldlegierung bevorzugt eine spezifische Wärmeleitfähigkeit von nicht mehr als 200 W/m*K auf.

Hohe Wärmeleitfähigkeiten von mehr als 100 W/m*K haben in Bezug auf die Anwendung der Elektrode als Teil einer Ablationsvorrichtung den Vorteil, dass kein aktives oder passives Kühlelement im Bereich der Katheter-Spitze für die Wärmeabfuhr benötigt wird. Dadurch verringert sich der konstruktive Aufwand der Ablationsvorrichtung beträchtlich. Eine Miniaturisierung der Elektrode und damit auch der Katheter-Vorrichtung wird dadurch vereinfacht. Weiterhin haben hohe thermische Leitfähigkeiten den Vorteil, dass das betroffene Gewebe effektiv, d.h. auf den betroffenen Bereich lokal begrenzt zerstört wird. Gesundes Gewebe, das an pathogenes Gewebe direkt angrenzt, wird dadurch nicht in unnötiger Weise mit zerstört.

In einer bevorzugten Ausführungsform der Ablationselektrode ist die Goldlegierung biokompatibel. Insbesondere eignet sich die Goldlegierung für den direkten Kontakt zu einem eukaryotischen Gewebe.

In einer bevorzugten Ausführungsform der Ablationselektrode weist die Goldlegierung eine spezifische elektrische Leitfähigkeit von wenigstens 15 m/Ω*mm² auf, bevorzugt von wenigstens 19 m/Ω*mm² auf, besonders bevorzugt von wenigstens 21 m/Ω*mm² auf. Dabei weist die Goldlegierung bevorzugt eine spezifische elektrische Leitfähigkeit von nicht mehr als 28 m/Ω*mm² auf.

In einer bevorzugten Ausführungsform der Ablationselektrode weist die Goldlegierung eine Vickers Härte HV 1 von wenigstens HV 1 = 100 auf, bevorzugt von wenigstens HV 1 = 150 auf, besonders bevorzugt von wenigstens HV 1 = 250 auf. Dabei weist die Goldlegierung bevorzugt eine Vickers Härte HV 1 von nicht mehr als 330 auf.

Die erfindungsgemäßen Ablationselektroden lassen sich mit Goldlegierungen mit vorgenannten Vickers Härten besonders einfach mit Hilfe von zerspanenden Verfahren auch in kleinen Geometrien, insbesondere mit geringen Wandstärken, herstellen. Zu hohe Vickers Härten, insbesondere von mehr als HV 1 = 330, können jedoch einen hohen Werkzeugverschleiß bedeuten und sind dadurch weniger bevorzugt.

In einer bevorzugten Ausführungsform der Ablationselektrode weist die Goldlegierung eine 0,2 %-Dehngrenze Rp_{0.2} von wenigstens Rp_{0.2} = 200 MPa auf, bevorzugt von wenigstens Rp_{0.2} = 500 MPa auf. Dabei weist die Goldlegierung bevorzugt eine 0,2 % Dehngrenze Rp_{0.2} von nicht mehr als 700 MPa auf.

Eine Mindestdehngrenze ist bevorzugt, um eine plastische Verformung des Werkstücks während der spanenden Bearbeitung zu vermeiden. Die plastische Verformung kann durch das Werkzeug oder die Halterung des Bauteils verursacht werden. Daher sind Ablationselektroden die Goldlegierungen mit minimaler 0,2 %-Dehngrenze von 200 MPa, insbesondere einer minimalen 0,2 %-Dehngrenze von 500 MPa aufweisen, besonders bevorzugt. 0,2 %-Dehngrenzen von mehr als 700 MPa können zu einem höheren Werkzeugverschleiß führen und sind daher weniger bevorzugt.

In einer bevorzugten Ausführungsform der Ablationselektrode weist die Goldlegierung eine Zugfestigkeit Rₘ von wenigstens Rₘ = 400 MPa auf, bevorzugt von wenigstens Rₘ = 600 MPa auf. Dabei weist die Goldlegierung bevorzugt eine Zugfestigkeit Rₘ von nicht mehr als 750 MPa auf.

Eine Mindestzugfestigkeit ist bevorzugt, um einen Bruch des Werkstücks durch den Druck während der spanenden Bearbeitung zu vermeiden. Daher sind Ablationselektroden die Goldlegierungen mit einer minimalen Zugfestigkeit von 400 MPa, insbesondere einer minimalen Zugfestigkeit von wenigstens 600 MPa, bevorzugt. Zugfestigkeiten von mehr als 750 MPa können zu einem höheren Werkzeugverschleiß führen und sind daher weniger bevorzugt.

In einer bevorzugten Ausführungsform der Ablationselektrode bilden die Elemente Gold und Kobalt in der Goldlegierung oberhalb von 800 °C einen einphasigen Mischkristall und die Legierung weist bei Zimmertemperatur und/oder bei Körpertemperatur eine Ausscheidung metastabiler Phasen auf.

In bevorzugter Weise bildet die Goldlegierung bei erhöhten Temperaturen einen einphasigen Mischkristall und bei rascher Abkühlung auf Zimmertemperatur eine zweite feinverteilte Ausscheidungs-Phase. Diese Phase ist auch bei Körpertemperatur stabil. Durch die vollständige Mischkristallbildung bei Temperaturen oberhalb von 800 °C kann das Verfahren der Bildung von Ausscheidungen auf effektive Art und Weise kontrolliert werden, da es bei der Abkühlung zu einer homogenen Verteilung der einzelnen Bereiche der Ausscheidungen im Gefüge der Matrix kommt. Dafür muss das Material aus der Mischkristallphase rasch abgekühlt (abgeschreckt) werden, um die bei hohen Temperaturen im Mischkristall vorhandenen Leerstellen im Kristall einzufrieren. Diese dienen als Keimzentren für die Ausscheidungen und ermöglichen eine feine Verteilung der Ausscheidungsphase.

In einer bevorzugten Ausführungsform weist die Ablationselektrode wenigstens teilweise die Grundform eines Zylinders auf, wobei der Zylinder wenigstens über einen Teil seiner Länge als Hohlzylinder ausgebildet ist, wobei der Hohlzylinder einen Außendurchmesser D und einen Innendurchmesser d aufweist.

Der Hohlzylinder kann bevorzugt derart ausgestaltet sein, dass (D-d)/2 eine Wandstärke dw definieren, wobei eine maximale Wandstärke d_{Wmax} nicht mehr als d_{Wmax} = 2 mm beträgt, bevorzugt nicht mehr als d_{Wmax} = 1 mm beträgt, besonders bevorzugt nicht mehr als d_{Wmax} = 0,5 mm beträgt. Dabei weist der Hohlzylinder bevorzugt eine minimale Wandstärke d_{Wmin} von wenigstens 0,08 mm auf.

Dabei ist die Ablationselektrode bevorzugt als Ringelektrode ausgestaltet, wobei die Ringelektrode die vorstehend genannten Wandstärken aufweist.

In einer weiteren bevorzugten Ausführungsform weist die Ablationselektrode die Grundform einer Halbkugel auf, wobei die Halbkugel einen Hohlraum aufweist. Die dadurch gebildete Halbkugel-Schale weist einen Außendurchmesser D und einen Innendurchmesser d auf.

Die Halbkugelschale kann bevorzugt derart ausgestaltet sein, dass (D-d)/2 eine Wandstärke dw definieren, wobei eine maximale Wandstärke d_{Wmax} nicht mehr als d_{Wmax} = 2 mm beträgt, bevorzugt nicht mehr als d_{Wmax} = 1 mm beträgt, besonders bevorzugt nicht mehr als d_{Wmax} = 0,5 mm beträgt. Dabei weist die Halbkugelschale bevorzugt eine minimale Wandstärke d_{Wmin} von wenigstens 0,08 mm auf.

Es sind auch Ausführungsformen denkbar, die eine Kombination aus Hohlzylinder und Halbkugelschale darstellen, beispielsweise eine Halbkugelschale die über die Stirnflächen mit einem Hohlzylinder verbunden ist.

Aufgrund der hohen thermischen und elektrischen Leitfähigkeit der erfindungsgemäßen Ablationselektrode ist die Fertigung von Elektroden mit geringer Wandstärke von weniger als 2 mm möglich, wobei Wandstärken bis 0,08 mm technisch realisierbar sind. Niedrige Wandstärken der Elektrode tragen dazu bei, dass der Durchmesser der Katheter-Spitze insgesamt verkleinert werden kann. Dadurch kann in Blutgefäßen der Eintritt in schwer zugängliche Bereiche erleichtert werden.

Einen Beitrag zur Erfüllung mindestens einer der erfindungsgemäßen Aufgaben leistet eine Katheter-Spitze zur Verwendung mit einer Katheter-Vorrichtung, umfassend eine Ablationselektrode nach einer der oben genannten Ausführungsformen.
In bevorzugter Weise wird die erfindungsgemäße Ablationselektrode als Teil einer Katheter-Spitze, die wiederum Teil einer Katheter-Vorrichtung am distalen Ende der Vorrichtung ist, eingesetzt.

In einer bevorzugten Ausführungsform der Katheter-Spitze weist die Ablationselektrode eine erste und eine zweite Oberfläche auf, wobei die erste Oberfläche mit einem elektrisch leitenden Element der Katheter-Vorrichtung verbunden ist, wenigstens die zweite Oberfläche die Goldlegierung umfasst und die zweite Oberfläche für einen Kontakt zu einem biologischen Gewebe bestimmt ist. Bevorzugt weist die Ablationselektrode die Grundform eines Zylinders auf, wobei der Zylinder wenigstens über einen Teil seiner Länge als Hohlzylinder ausgebildet ist der einen Außendurchmesser D und einen Innendurchmesser r aufweist, wobei (D-d)/2 eine Wandstärke dw definieren. Bevorzugt bildet die Außenseite des Zylinders und/oder Hohlzylinders wenigstens teilweise die zweite Oberfläche, die für den Kontakt mit einem biologischen Gewebe bestimmt ist. Bevorzugt bildet die Innenwandung des Hohlzylinders wenigstens teilweise die erste Oberfläche, die mit einem elektrisch leitenden Element der Katheter-Vorrichtung verbunden ist.

Vorzugsweise weist nur der äußere Bereich der Elektrode, der einen Kontakt zum zu zerstörenden Gewebe während der Behandlung herstellt, die erfindungsgemäße Goldlegierung auf. Auf der Innenseite kann zur Übertragung der elektrischen Signale und zum Wärmetransport eine andere Legierung verwendet werden.

In einer weiteren Ausführungsform ist auch denkbar, dass die Ablationselektrode bzw. die Katheter-Spitze nur in denjenigen Bereichen die Goldlegierung aufweist, in denen eine besonders hohe Wärmeübertragung erforderlich ist.

In einer bevorzugten Ausführungsform weist die Katheter-Spitze kein weiteres Kühlelement außer der Ablationselektrode aufweist.

Die Ablationselektrode selbst kann aufgrund ihrer hohen Wärmeleitfähigkeit als Kühlelement aufgefasst werden, da nicht nur Wärme zum Gewebe transportiert sondern auch Wärme aus dem Gewebe über die Ablationselektrode abgeführt wird. Von der Ablationselektrode abgesehen ist die Katheter-Spitze somit kühlelementfrei. Bei dem Kühlelement kann es sich um ein aktives oder passives Kühlelement handeln. Wenn im Bereich der Katheter-Spitze keine zusätzlichen aktiven und/oder passiven Kühlelemente verbaut werden wird der konstruktive Aufbau der Katheter-Spitze insgesamt vereinfacht. Unter einem passiven Kühlelement wird beispielsweise eine Vorrichtung verstanden, die es ermöglicht ein Kühlmedium kontinuierlich durch das Kühlelement zu fördern. Ein passives Kühlelement kann beispielsweise ein weiteres metallisches Element mit einer hohen Wärmeleitfähigkeit beinhalten, das jedoch nicht am eigentlich Ablationsprozess beteiligt ist, das heißt keine Hochfrequenzenergie an das Gewebe überträgt.

Einen Beitrag zur Erfüllung mindestens einer der erfindungsgemäßen Aufgaben leistet eine Katheter-Vorrichtung für die Hochfrequenzablation, umfassend eine Katheter-Spitze nach einer der vorherigen Ausführungsformen.

In bevorzugter Weise wird die erfindungsgemäße Katheter-Spitze als Teil einer Katheter-Vorrichtung eingesetzt. Die Katheter-Vorrichtung findet bevorzugt Anwendung in der Hochfrequenzablation.

In einer bevorzugten Ausführungsform umfasst die Katheter-Vorrichtung einen langgestreckten und wenigstens teilweise flexiblen Katheter-Körper, wobei der Katheter-Körper ein proximales und ein distales Ende aufweist und sich die Ablationskatheter-Spitze im Bereich des distalen Endes des Katheter-Körpers befindet.

Im Bereich des distalen Endes des Katheter-Körpers findet die eigentliche Behandlung, d.h. die Zerstörung des pathogenen Gewebes statt, während das proximale Ende mit der Steuervorrichtung der Katheter-Vorrichtung verbunden ist.

Einen Beitrag zur Erfüllung mindestens einer der erfindungsgemäßen Aufgaben leistet die Verwendung einer Ablationselektrode nach einer der vorherigen Ausführungsformen, die Verwendung einer Katheter-Spitze nach einer der vorherigen Ausführungsformen oder die Verwendung der Katheter-Vorrichtung nach einer der vorherigen Ausführungsformen für die Hochfrequenzablation.

Einen Beitrag zur Erfüllung mindestens einer der erfindungsgemäßen Aufgaben leistet ein Verfahren zur Herstellung einer Ablationselektrode, wobei das Verfahren folgende Schritte beinhaltet:
a. Bereitstellen eines Vorläufers aus einer Goldlegierung, wobei die Goldlegierung wenigstens 80 Gew.-% Gold aufweist und 1 Gew.-% bis 9 Gew.-% Kobalt aufweist.
b. Formen des Vorläufers zu einem drahtförmigen Formkörper;
c. Lösungsglühen des Formkörpers bei einer Temperatur von 800 bis 1000 °C für wenigstens 15 min.
d. Abschrecken des lösungsgeglühten Formkörpers durch Eintauchen in ein flüssiges Medium, so dass der Formkörper um mehr als 500 °C abkühlt.
e. Auslagerungsglühen des abgeschreckten Formkörpers bei einer Temperatur von 200 °C bis 600 °C für eine Dauer von wenigstens 0,5 h.
f. Formen des auslagerungsgeglühten Formkörpers zu der Ablationselektrode.

Zur Erzielung hoher elektrischer und thermischer Leitfähigkeiten der erfindungsgemäßen Ablationselektrode ist es erforderlich, die Goldlegierung einer Ausscheidungshärtung zu unterziehen.

Dabei wird in Schritt a. zunächst ein Vorläufer der Goldlegierung mit den genannten Gewichtsanteilen Kobalt bereitgestellt, beispielsweise durch Schmelzen der Ausgangsmaterialien im Strangguss-Verfahren mit Hilfe eines Induktionsofens, wobei vorzugsweise Formkörper mit kreisförmigem Querschnitt erhalten werden.

Anschließend wird dieser Vorläufer in Schritt b. zu einem drahtartigen Formkörper umgeformt, was beispielsweise mit Hilfe mehrerer Ziehsteine in mehreren Ziehstufen realisiert werden kann. Die erhaltenen drahtartigen Formkörper werden gerichtet und auf die passende Länge zugeschnitten.

Anschließend werden die erhaltenen Formkörper gemäß Schritt c. lösungsgeglüht, wobei vorzugsweise eine einphasige Legierung mit homogener Mischkristallbildung erhalten wird. Die Dauer des Lösungsglühens hängt von der Größe des Werkstücks ab. Üblicherweise wird das Lösungsglühen für wenigstens 15 min und höchstens 3 h durchgeführt. Längere Glühzeiten haben jedoch keinen nachteiligen Effekt.

Beim anschließenden Abschrecken des lösungsgeglühten Formkörpers gemäß Schritt d. und dem anschließenden Auslagerungsglühen gemäß Schritt e. wandelt sich der einphasige Mischkristall in eine Zweiphasenlegierung um.

Das Abschrecken gemäß Schritt d. wird derart durchgeführt, dass sich der Formkörper um einen Betrag von wenigstens 500 °C abkühlt. Vorzugsweise wird der Formkörper um einen Beitrag von wenigstens 700 °C abgekühlt, weiter bevorzugt erfolgt die Abkühlung auf Zimmertemperatur.

Um den gewünschten Effekt der Ausscheidungshärtung zu erzielen, das heißt um die vorteilhaften mechanischen Eigenschaften und eine hohe elektrische und thermische Leitfähigkeit zu erhalten, muss das anschließende Auslagerungsglühen gemäß Schritt e. für wenigstens 0,5 h durchgeführt werden. Üblicherweise wird das Auslagerungsglühen für höchstens 40 h durchgeführt, längere Zeiten für das Auslagerungsglühen müssen jedoch keinen nachteiligen Effekt haben.

Die mit a. bis f. bezeichneten Verfahrensschritte werden bevorzugt in der genannten Reihenfolge durchgeführt.

In einer alternativen Ausführung werden nach Bereitstellen des Vorläufers gemäß Schritt a. anschließend die Schritte c. bis e. durchgeführt. Nach dem Auslagerungsglühen gemäß Schritt e. erfolgt das Formen des ausscheidungsgehärteten Materials zu einem drahtartigen Formkörper und anschließend das Formen zur Ablationselektrode. Dies ist jedoch nur dann möglich, wenn die verwendeten Umformmaschinen, beispielsweise Drahtzüge oder Walzen, das Material aufgrund der durch das Auslagerungsglühen erzielten hohen Festigkeit umformen können, ohne dass es zu Abrissen oder anderen fertigungstechnischen Problemen kommt.

In einer bevorzugten Ausführungsform des Verfahrens weist die Goldlegierung 1 Gew.-% bis 9 Gew.-% Kobalt und mindestens 90 Gew.-% Gold auf.

In einer bevorzugten Ausführungsform des Verfahrens weist die Goldlegierung 2 Gew.-% bis 8 Gew.-% Kobalt auf, besonders bevorzugt weist die Goldlegierung 4 Gew.-% bis 6 Gew.-% Kobalt auf und weiter bevorzugt weist die Goldlegierung 5 Gew.-% Kobalt auf.

In einer bevorzugten Ausführungsform des Verfahrens findet das Lösungsglühen gemäß Schritt c. und/oder das Auslagerungsglühen gemäß Schritt e. unter reduzierender Atmosphäre statt.

In einer bevorzugten Ausführungsform des Verfahrens findet das Lösungsglühen gemäß Schritt c. und/oder das Auslagerungsglühen gemäß Schritt e. unter einer Inertgasatmosphäre statt.

In einer bevorzugten Ausführungsform des Verfahrens beinhaltet die reduzierende Atmosphäre etwa 95 Vol.-% Stickstoff und etwa 5 Vol.-% Wasserstoff.

Kobalt bildet leicht die Oxide CoO (Kobalt(II)-oxid), Co₂O₃ (Kobalt(III)-oxid) und Co₃O₄ (Kobalt(II,III)-oxid). Um diese Oxidation zu verhindern, wird das Lösungsglühen bevorzugt unter einer Inertgasatmosphäre oder unter einer reduzierenden Atmosphäre durchgeführt.

In einer bevorzugten Ausführungsform des Verfahrens wird das Auslagerungsglühen gemäß Schritt e. für eine Dauer von 15 h bis 30 h durchgeführt, besonders bevorzugt für eine Dauer von 17 h bis 30 h durchgeführt, weiter bevorzugt für eine Dauer von 24 h bis 30 h durchgeführt. Speziell bevorzugt beträgt die Dauer des Auslagerungsglühens 30 h.

Im Hinblick auf die Erzielung besonders hoher elektrischer und thermischer Leitfähigkeiten hat sich gezeigt, dass die Auslagerungsglühung bevorzugt für wenigstens 15 h durchgeführt werden muss. Besonders gute Ergebnisse werden mit einer Glühdauer von 30 h erzielt. Ähnlich gute Ergebnisse sind bei einer Glühdauer im Bereich zwischen 25 h und 40 h zu erwarten.

In einer bevorzugten Ausführungsform des Verfahrens erfolgt das Abschrecken des Formkörpers nach Schritt d. durch Eintauchen in ein Wasserbad.

Das Abschrecken kann in besonders einfacher Weise, ohne hohen apparativen Aufwand, mit Hilfe eines Wasserbades erfolgen.

Einen Beitrag zur Erfüllung mindestens einer der erfindungsgemäßen Aufgaben leistet ein Verfahren nach wenigstens einer der vorherigen Ausführungsformen zur Herstellung einer Ablationselektrode nach wenigstens einer der vorherigen Ausführungsformen.

Bevorzugte Ausgestaltungen von Bestandteilen einer erfindungsgemäßen Kategorie, insbesondere der erfindungsgemäßen Ablationselektrode, der erfindungsgemäßen Katheter-Spitze, der erfindungsgemäßen Katheter-Vorrichtung, der erfindungsgemäßen Verwendung und des erfindungsgemäßen Verfahrens, sind ebenso bevorzugt für gleichnamige oder entsprechende Bestandteile der jeweils anderen erfindungsgemäßen Kategorie.

### DEFINITIONEN

### Ablationselektrode, Katheter-Spitze, Katheter-Körper, Katheter-Vorrichtung

Eine Ablationselektrode ist Bestandteil einer Katheter-Spitze. Die Katheter-Spitze ist Bestandteil eines Katheter-Körpers einer Katheter-Vorrichtung. Der Katheter-Körper ist wenigstens im Bereich der Katheter-Spitze flexibel ausgestaltet.

Die Katheter-Vorrichtung weist ein proximales und ein distales Ende auf. Am distalen Ende befindet sich die Katheter-Spitze der Katheter-Vorrichtung.

Der Katheter-Körper weist ein proximales und ein distales Ende auf. Am distalen Ende befindet sich die Katheter-Spitze der Katheter-Vorrichtung.

Die Katheter-Spitze wiederum umfasst eine oder mehrere Ablationselektroden. Die Ablationselektrode oder die Ablationselektroden transportieren Wärmeenergie zum Gewebe.

Die Katheter-Vorrichtung ist in Verbindung mit der Katheter-Spitze und der Ablationselektrode oder den Ablationselektroden für alle denkbaren elektrophysiologischen medizinischen Anwendungen geeignet. Dazu zählt insbesondere die Hochfrequenzablation. Weitere mögliche Anwendungen sind Kryoablation und diagnostische Anwendungen mit Mapping-Kathetern. Sämtliche Materialien der Ablationselektrode, der Katheter-Spitze, des Katheter-Körpers und der Katheter-Vorrichtungen sind vorzugsweise biokompatibel. Insbesondere besteht die Ablationselektrode ausschließlich aus biokompatiblen Metallen und/oder einer biokompatiblen Legierung.

### Biokompatibles Metall, biokompatible Legierung

Ein bevorzugtes biokompatibles Metall und/oder eine biokompatible Legierung ist biotolerant und/oder bioinert. Insbesondere ist ein bevorzugtes Metall und/oder eine bevorzugte Legierung als biokompatibel zertifizierbar im Sinne der ISO 10993 1-20 und eignet sich für den direkten Kontakt zu eukaryotischem Gewebe.

### Ausscheidung metastabiler Phasen

Im Rahmen der Erfindung bedeutet "Ausscheidung metastabilier Phasen", dass im elementaren Mischkristall einer Legierung eine Ausscheidung in feinverteilter Form stattfand. Diese Ausscheidung bewirkt insbesondere ein Hindernis für Versetzungsbewegungen und dadurch eine Erhöhung der Festigkeit. Insbesondere wird die Ausscheidung metastabiler Phasen durch Ausscheidungshärtung bewirkt.

### Zimmertemperatur

Unter Zimmertemperatur wird im Rahmen der Erfindung eine Temperatur verstanden, die üblicherweise in einem geschlossenen Raum herrscht, üblicherweise 15 °C bis 30 °C, vorzugsweise 20 bis 25 °C.

### Körpertemperatur

Unter Körpertemperatur wird im Rahmen der Erfindung eine Temperatur verstanden, die während einer Katheter-Ablation eines menschlichen oder tierischen Körpers zur Zeit der Behandlung im Körperinneren herrscht. Insbesondere ist die Körpertemperatur die Normaltemperatur eines gesunden menschlichen oder tierischen Körpers, wobei diese Temperatur in einem Bereich von 36,3 °C bis 37,4 °C liegt. Es versteht sich dass auch niedrigere oder höhere Körpertemperaturen denkbar sind, beispielsweise im Falle einer Unterkühlung oder im Falle einer mit Fieber verbundenen Erkrankung.

### MESSMETHODEN

### Spezifische elektrische Leitfähigkeit

Die elektrische Leitfähigkeit der jeweiligen Materialien wird gemäß DIN IEC 60468 mittels einer Vierpunktmessung an Drähten bestimmt. Im Messaufbau werden Drähte mit einer effektiven Prüflänge von 240 mm beziehungsweise 1000 mm Länge elektrisch leitend eingespannt. Ein konstanter elektrischer Strom geeigneter Stromstärke, hier 1000 mA, wird über eine Drahtlänge von 400 mm beziehungsweise 1300 mm im Draht eingespeist. Die Differenz der Länge von Stromeinspeisung und Spannungsmessung dient zur Ausbildung einer einheitlichen Stromdichte über die Prüflänge. Die über die Prüflänge abfallende elektrische Spannung wird gemessen. Aus den erhaltenen Spannungs-Werten wird die spezifische elektrische Leitfähigkeit mit Hilfe des Ohm'schen Gesetzes bestimmt. Die Bestimmung des tatsächlichen Drahtdurchmessers erfolgt mit Hilfe einer Mikrometer-Schraube. Die Prüflänge wird mit der Einspannvorrichtung der Drähte bestimmt.

Die Einspannvorrichtung ist vom Typ BURSTER Typ 2381 "precision clamping device" für Vierpolmessungen. Als Spannungsmessgerät wird ein Keithley Multimeter 2000 verwendet. Als Stromquelle dient eine TTi PL310 power supply unit. Die Temperatur während der Messung beträgt zwischen 20 °C und 22 °C.

### Spezifische Wärmeleitfähigkeit

Die spezifische Wärmeleitfähigkeit wird indirekt durch Umrechnung aus der experimentell bestimmten spezifischen elektrischen Leitfähigkeit nach dem Wiedemann-Franz Gesetz ermittelt.

### Vickers Härte

Die Härte wird mit Hilfe eines Härteprüfgeräts Typ VMHT MOT von Leica nach Vickers gemäß der DIN EN ISO 6507-1 bestimmt. Ein pyramidenförmiger Prüfkörper wird dabei mit einer definierten Last senkrecht in die metallographisch polierte Materialoberfläche eingedrückt. Aus dem Querschnitt des vom Prüfkörper hinterlassenen Eindrucks und der Prüflast wird die Härte berechnet. Die Prüflast beträgt 1 kg und wird folglich in der Einheit HV1 angegeben. Die Temperatur während der Messung beträgt zwischen 20 °C und 22 °C.

### 0,2 % Dehngrenze Rp_{0.2} und Zugfestigkeit Rₘ

Die Dehngrenze und die Zugfestigkeit wird jeweils in einem dehnungsgesteuerten Zugversuch gemäß DIN ISO 6892-1 bestimmt. Als Anlage dient eine Universalprüfmaschine Zwick Roell, Typ Z250/SN5A. Als Kraftmesseinrichtung wird eine 200 kN-Kraftmessdose mit einem Messbereich von 0,4 bis 240 kN verwendet. Als Längenänderungs-Aufnehmer dient eine Vorrichtung der Firma Multisense mit einem Messbereich von 50 mm. Die Probenlänge des Drahtes beträgt 200 mm bei einer Einspannlänge von 100 mm und einer effektiven Prüflänge von 50 mm. Die Prüfgeschwindigkeit beträgt 10 mm pro Minute. Als Werkzeug für die Probeneinspannung dient ein Zwick 8406 Spannwerkzeug.

### BEISPIELE

Die Erfindung wird im Folgenden durch Beispiele und Zeichnungen genauer dargestellt, wobei die Beispiele und Zeichnungen keine Einschränkung der Erfindung bedeuten. Die Zeichnungen sind, sofern nicht anders angegeben, nicht maßstabsgetreu.

### Erfindungsgemäßes Beispiel

Es wurden drahtartige Formkörper mit Durchmessern zwischen 3,0 und 3,2 mm hergestellt, die sich zur Herstellung erfindungsgemäßer Ablationselektroden eignen. Die mechanischen und physikalischen Eigenschaften wurden anhand von diesen drahtartigen Formkörpern bestimmt. Anschließend wurden die getesteten Formkörper mit Hilfe spanender Verfahren zu Ablationselektroden mit einer Basis-Wandstärke von 0,29 mm weiterverarbeitet. An diesen Ablationselektroden wurden zusätzlich Bohrungen vorgenommen. Im Bereich der durch die Bohrungen erzeugten Vertiefungen betrug die Wandstärke 0,16 mm.

Zunächst wurde ein Vorläufer für eine Goldlegierung mit einem Gewichtsanteil von 95,0 Gew.-% Gold und 5,0 Gew.-% Kobalt durch Schmelzen im Strangguss-Induktionsofen hergestellt. Als Ansatzmaterialien wurden Gold-Granulat mit einer Reinheit von 99,99 Gew.-% und Kobalt in Stücken mit einer Reinheit von 99,9 Gew.-% verwendet. Das Gold-Granulat und die Kobalt-Stücke wurden in einem Ansatz von 2400 g in einem Tontiegel unter Kohlenmonoxid-Atmosphäre vorgeschmolzen. Anschließend wurde die Schmelze in eine Grafitform in Barren mit einer Kantenlänge von 20x20 mm abgegossen. Nach Erkalten lassen auf Zimmertemperatur wurde die Gold-Kobalt-Legierung bei 1100 °C umgeschmolzen und im Strangguss-Verfahren abgegossen. Dadurch wurden Formkörper mit rundem Querschnitt, einem Durchmesser von 7 mm und einer Länge von 3400 mm erhalten. Anschließend wurde die chemische Zusammensetzung der Goldlegierung per Glimmentladungsspektroskopie analysiert. Die dabei bestimmte Zusammensetzung betrug 95,4 Gew.-% Gold und 4,6 Gew.-% Kobalt.

Der so erhaltene Vorläufer der Goldlegierung wurde mit Hilfe einer Malmedie-Einzelziehanlage mit Diamant-Ziehsteinen durch Drahtziehen im Querschnitts-Durchmesser von 7,0 mm auf einen Solldurchmesser von 3,0 mm reduziert. Das Drahtziehen erfolgte über mehrere Zwischen-Ziehstufen, ausgehend vom Vorläufer mit 7,0 mm, über 6,00 mm, 5,00 mm, 4,20 mm, 3,70 mm, 3,40 mm und 3,10 mm auf 3,00 mm. Die Ziehgeschwindigkeit betrug 15 m pro Minute. Der erhaltene drahtartige Formkörper wurde gerichtet und zu Stäben mit jeweils 1800 mm Länge geschnitten. Es wurden Drahtstücke mit einem ist-Durchmesser zwischen 3,0 mm und 3,20 mm erhalten.

Die drahtartigen Formkörper wurden anschließend einer Lösungsglühung unterzogen. Dafür wurden die Formköper in einen Rohrofen (Hersteller: Carbolite® Gero, maximale Temperatur. 1200 °C) eingebracht und für eine Stunde bei 950 °C unter Formiergas (Zusammensetzung: 95 Vol.-% Sticktstoff, 5 Vol.-% Wasserstoff) geglüht. Die Formkörper wurden aus dem heißen Ofen entnommen und anschließend sofort durch Eintauchen in ein Wasserbecken abgeschreckt.

Anschließend wurde in oben genanntem Rohrofen für die Ausscheidungshärtung eine Auslagerungsglühung bei 400 °C pro zu testendem Formkörper unter Formiergas durchgeführt. Gemäß Beispiel 1 wurde die Auslagerungsglühung für 10 h durchgeführt. Gemäß Beispiel 2 wurde die Auslagerungsglühung für 17 h durchgeführt. Gemäß Beispiel 3 wurde die Auslagerungsglühung für 24 h durchgeführt und gemäß Beispiel 4 wurde die Auslagerungsglühung für 30 h durchgeführt.

### Nicht erfindungsgemäße Vergleichsbeispiele

Als Vergleichsbeispiele wurden drahtartige Formkörper getestet, die wie in den Beispielen über einen Querschnitts-Durchmesser von 3,0 bis 3,2 mm verfügten.

Als Vergleichsbeispiel 1 wurde ein drahtartiger Formkörper aus einer Goldlegierung mit einem Anteil von 95 Gew.-% Gold und 5 Gew.-% Kobalt getestet, der keiner Auslagerungsglühung und damit keiner Ausscheidungshärtung unterzogen wurde. Als Vergleichsbeispiel 2 wurde eine Goldlegierung mit einem Anteil von 99 Gew.-% Gold und 1 Gew.-% Titan getestet. Dieses Legierungssystem ist für eine Ausscheidungshärtung zugänglich und wurde für 1 h bei 550 °C einer Auslagerungsglühung unterzogen. Für dieses System wurde in Versuchen die größte Härte bei1 h Glühzeit (Auslagerungsglühung) erhalten. Als Vergleichsbeispiel 3 wurde ein drahtartiger Formkörper aus reinem Gold getestet. Als Vergleichsbeispiel 4 wurde eine Goldlegierung mit 90 Gew.-% Gold und 10 Gew.-% Platin getestet. Als Vergleichsbeispiel 5 wurde eine Platin-Iridium-Legierung mit einem Anteil von 90 Gew.-% Platin und 10 Gew.-% Iridium getestet. Als Vergleichsbeispiel 6 wurde eine Palladium-Platin-Legierung mit einem Anteil von 80 Gew.-% Pallladium und 20 Gew.-% Platin getestet. Die Legierungssysteme der Vergleichsbeispiele 4 bis 6 sind keiner Ausscheidungshärtung zugänglich und wurden daher keiner Auslagerungsglühung unterzogen.

### Auswertung

Die Beispiele der nachfolgenden Tabelle zeigen die thermischen und elektrischen Leitfähigkeiten der durch Ausscheidungshärtung erhaltenen Formkörper der Beispiele 1 bis 4 verglichen mit nicht erfindungsgemäßen Vergleichsbeispielen. Die angegebene Zeit in Stunden im Falle der erfindungsgemäßen Formkörper aus AuCo5 ist die Dauer der Auslagerungsglühung und dadurch die Dauer der Ausscheidungshärtung.

| **Beispiel Nr.** | **Zusammensetzung Formkörper** | **Thermische Leitfähigkeit / (W/m*K)** | **Elektrische Leitfähigkeit / (m/Ω*mm²)** |
|---|---|---|---|
| Beispiel 1 | AuCo5, 10 h | 110,2 | 15,2 |
| Beispiel 2 | AuCo5, 17 h | 142,9 | 19,7 |
| Beispiel 3 | AuCo5, 24 h | 142,0 | 19,6 |
| Beispiel 4 | AuCo5, 30 h | 156,4 | 21,6 |
| Vergleichsbeispiel 1 | AuCo5 | 14,0 | 1,9 |
| Vergleichsbeispiel 2 | AuTi1 | 30,0 | 4,2 |
| Vergleichsbeispiel 3 | Au (99,99 Gew.-%) | 317,5 | 43,9 |
| Vergleichsbeispiel 4 | AuPt10 | 72,5 | 10,0 |
| Vergleichsbeispiel 5 | PtIr10 | 29,0 | 4,0 |
| Vergleichsbeispiel 6 | PdPt20 | 36,3 | 5,0 |

Die Tabelle zeigt die verbesserte thermische und elektrische Leitfähigkeit ausscheidungsgehärteter AuCo5 Formkörper gegenüber Formkörpern aus herkömmlichen nicht ausscheidungshärtbaren Legierungen auf Basis von AuPt, Ptlr und PdPt. Weiterhin wird der vorteilhafte Effekt der Ausscheidungshärtung gegenüber einer nicht ausscheidungs-gehärteten AuCo5 Legierung gezeigt. Ferner zeigt die vorstehende Tabelle die deutlich höhere thermische und elektrische Leitfähigkeit ausscheidungsgehärteter Formkörper auf Gold-Kobalt Basis gegenüber ausscheidungsgehärteten Formkörpern auf Basis von Gold-Titan.

Die verbesserten mechanischen Eigenschaften der auf Basis von AuCo5 hergestellten Formkörper gegenüber Formkörpern insbesondere auf Basis von reinem Gold zeigt die folgende Tabelle. Gezeigt werden die 0,2 % Dehngrenze Rp_{0,2}, die Zugfestigkeit Rₘ und die Vickers Härte HV 1.

| **Beispiel Nr.** | **Zusammensetzung Formkörper** | **Rp_{0,2} / MPa** | **Rₘ / MPa** | **HV 1** |
|---|---|---|---|---|
| Beispiel 1 | AuCo5, 10 h | 589 | 663 | 259,6 |
| Beispiel 2 | AuCo5, 17 h | 216 | 469 | 176,0 |
| Beispiel 3 | AuCo5, 24 h | 218 | 504 | 106,3 |
| Beispiel 4 | AuCo5, 30 h | 297 | 434 | 132,3 |
| Vergleichsbeispiel 1 | AuCo5 | nicht bestimmt | nicht bestimmt | 142,4 |
| Vergleichsbeispiel 2 | AuTi1 | 430 | 559 | 171,9 |
| Vergleichsbeispiel 3 | Au (99,99 Gew.-%) | 217 | 221 | 75,7 |
| Vergleichsbeispiel 4 | AuPt10 | 409 | 451 | nicht bestimmt |
| Vergleichsbeispiel 5 | PtIr10 | 600 | 640 | 200 |
| Vergleichsbeispiel 6 | PdPt20 | 545 | 578 | 60 |

Insbesondere die für 10 h ausscheidungsgehärtete AuCo5 Legierung zeigt gute mechanische Eigenschaften bezüglich der Dehngrenze und der Zugfestigkeit. Dort wo die mechanischen Kenndaten der ausscheidungsgehärteten AuCo5 vergleichbar mit den nicht ausscheidungsgehärteten Legierungssystemen AuPt5, AuPt10, PtIr10 und PdPt20 sind, werden entsprechend obiger Tabelle jedoch bessere Leitfähigkeiten erzielt. So zeigt beispielsweise AuPt10 bezüglich der Dehngrenze bessere Eigenschaften verglichen mit 17 h oder länger ausscheidungsgehärtetem AuCo5, weist jedoch deutlich niedrigere Leitfähigkeiten aus.

Zusammenfassend lässt dich daher feststellen dass Ablationselektroden aus ausscheidungsgehärteter Gold-Kobalt-Legierung mit der erfindungsgemäßen Zusammensetzung optimale Eigenschaften hinsichtlich der Kombination der Merkmale elektrische und thermische Leitfähigkeit, Dehngrenze, Zugfestigkeit und Härte aufweisen und dadurch besser für Ablationsanwendungen geeignet sind und gleichzeitig leichter herzustellen sind als herkömmliche Legierungen auf Basis von AuPt, Ptlr, PdPt oder reinem Gold.

### Herstellung von Spitzen-Elektroden aus AuCo5

Auf Basis der getesteten Formkörper wurden für Ablationsanwendungen geeignete Spitzen-Elektroden 103 hergestellt. Dafür wurden Drahtstücke mit einem Querschnitts-Durchmesser von 3,0 mm und einer Länge von 400 mm zu Spitzen-Elektroden mit einer Wandstärke von 0,29 mm bzw. 0,16 mm im Bereich der Bohrungen umgeformt. Zur Umformung des Drahtes zur Spitzen-Elektrode wurde eine mehrachsige Langdrehmaschine verwendet und die Spitzen-Elektrode mit entsprechend geeigneten Dreh- und Bohrwerkzeugen spanend gefertigt.

Es zeigen:
- Figur 1: eine Draufsicht einer Katheter-Vorrichtung umfassend eine Katheter-Spitze mit mehreren Ablationselektroden;
- Figur 2: einen Querschnitt durch einen Abschnitt einer Katheter-Spitze;
- Figur 3: eine perspektivische Ansicht a) und einen Querschnitt b) durch eine erfindungsgemäße Ablationselektrode, ausgestaltet als Ringelektrode;
- Figur 4: ein Ablaufdiagramm für ein erfindungsgemäßes Verfahren.

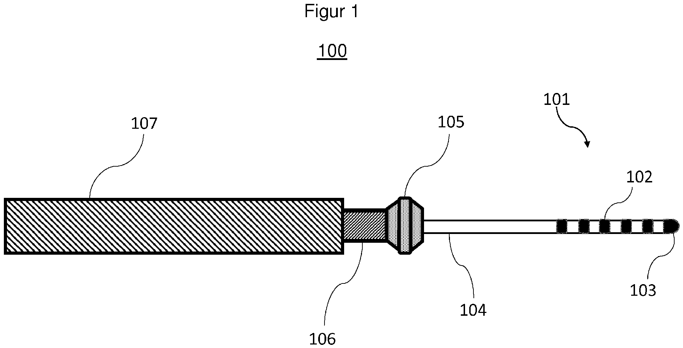

Figur 1 zeigt eine schematische Draufsicht einer Katheter-Vorrichtung 100, die mehrere erfindungsgemäße Ablationselektroden 102, 103 umfasst. Die Ablationselektroden 102, 103 sind als Ringelektroden 102 und Spitzenelektrode 103 ausgestaltet. Die gesamte Katheter-Vorrichtung 100 ist für Ablationsanwendungen geeignet. Die Katheter-Vorrichtung 100 weist einen langgestreckten Katheter-Körper 104 auf. Der Katheter-Körper 104 umfasst ein proximales und ein distales Ende. Das proximale Ende des Katheter-Körpers 104 ist mit einer Daumenauflage 105 verbunden, die wiederum mit einem beweglichen Kolben 106 verbunden ist. Der bewegliche Kolben 106 ist mit einem Steuergriff 107 verbunden. Im Bereich des distalen Endes des Katheter-Körpers 104 befindet sich die Katheter-Spitze 101, die flexibel ausgestaltet ist. Im Bereich der Katheter-Spitze 101 sind mehrere Ringelektroden 102 als Ablationselektroden 102, 103 angeordnet, wobei die Ringelektroden 102 den Katheter-Körper 104 vollständig umschließen. Am distalen Ende der Katheter-Spitze 101 befindet sich eine einen inneren Hohlraum aufweisende Spitzenelektrode 103 als weitere Ablationselektrode 102, 103.

Die Ringelektroden 102 als auch die Spitzenelektrode 103 sind bevorzugt aus einer Gold-Kobalt Legierung mit einem Gewichts-Anteil von 95 % Gold und 5 % Kobalt aufgebaut. Die Länge der Ringelektroden 102 beträgt in der Regel zwischen 3 und 6 mm. Die einzelnen Ringelektroden 102 weisen vorzugsweise einen gleichen Abstand zueinander auf. Der Abstand zwischen den einzelnen Ringelektroden 102 beträgt jeweils wenigstens 2 mm, vorzugsweise 3 bis 4 mm. Bei zu kleinem Abstand weist die Katheter-Spitze 101 eine zu geringe Flexibilität auf. Die Spitzenelektrode 103 weist eine Länge zwischen 2 und 5 mm auf. Die Ringelektroden 102 sowie die Spitzenelektrode 103 weisen bevorzugt eine Wandstärke von 0,1 bis 0,3 mm auf. Die Ringelektroden 102 sowie die Spitzenelektroden 103 sind sowohl bioinert als auch biokompatibel.

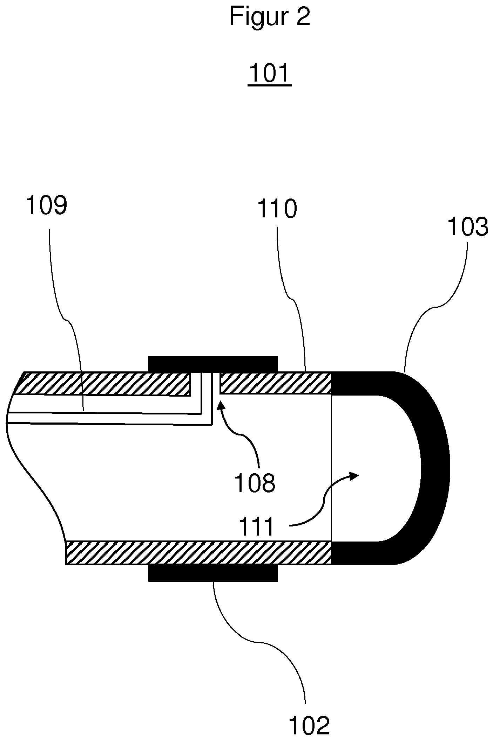

Figur 2 zeigt einen Querschnitt durch einen Abschnitt der Katheter-Spitze 101. Dargestellt ist der Abschnitt umfassend die Spitzenelektrode 103 und eine Ringelektrode 102. Die Spitzenelektrode 103 hat eine insgesamt halbkugelförmige Gestalt, weist einen Hohlraum 111 auf und kann an dem distalen Ende des Katheter-Körpers 104 an dessen Wandung 110 durch geeignete Mittel, beispielsweise durch einen Klebstoff, befestigt sein (nicht gezeigt). Jede der Elektroden 102 und 103 ist mit einer separaten elektrischen Leitung verbunden. Gezeigt ist die elektrische Leitung 109, die durch eine Ausnehmung 108 in der Wandung 110 des Katheter-Körpers verläuft und einen elektrischen Kontakt zwischen der Ringelektrode 102 und der Steuereinheit (nicht gezeigt) der Katheter-Vorrichtung 100 herstellt. Die Leitung 109 ist als Draht ausgebildet und erstreckt sich ausgehend von der Ringelektrode 102 durch den Innenraum des Katheter-Körpers 104 bis zum Steuergriff 107 und weiter zur Steuereinheit (nicht gezeigt). Die Leitung ist vorzugsweise ein Draht mit hoher elektrischer Leitfähigkeit, beispielsweise ein Kupferdraht. Die Leitung 109 überträgt die für die Behandlung erforderliche Hochfrequenz-Energie von der Steuereinheit aus an die als Ringelektroden 102 und Spitzen-Elektrode 103 ausgestalteten Ablationselektroden 102, 103. Die Ringelektroden 102 und die Spitzen-Elektrode 103 weisen vorzugsweise eine Goldlegierung mit einem Gewichtsanteil von 95 Gew.-% Gold und 5 Gew.-% Kobalt auf. Dabei kann die Ringelektrode 102 und/oder die Spitzen-Elektrode 103 im Bereich der zum Katheter-Körper 104 hin nach innen gerichteten Fläche gegebenenfalls aus einer anderen Legierung bestehen. In diesem Fall sind die Ringelektroden 102 und/oder die Spitzenelektrode 103 aus zwei unterschiedlichen Legierungen aufgebaut. Ein solcher Aufbau kann beispielsweise durch mechanische Bearbeitung eines Kern-Mantel-Drahtes erreicht werden, wobei der Mantel vorzugsweise eine Gold-Kobalt-Legierung mit erfindungsgemäßer Zusammensetzung aufweist. Vorzugsweise weist der Katheter-Körper 104 keine aktive und/oder passive Kühleinrichtung auf, ist also kühlelementfrei. Aufgrund der durch die verwendete Goldlegierung hohen Wärmeleitfähigkeit der als Ringelektroden 102 und Spitzen-Elektrode 103 ausgestalteten Ablationselektroden 102, 103 wird die bei der Behandlung erzeugte Wärme auch ohne aktive und/oder passive Kühleinrichtung effektiv abgeführt.

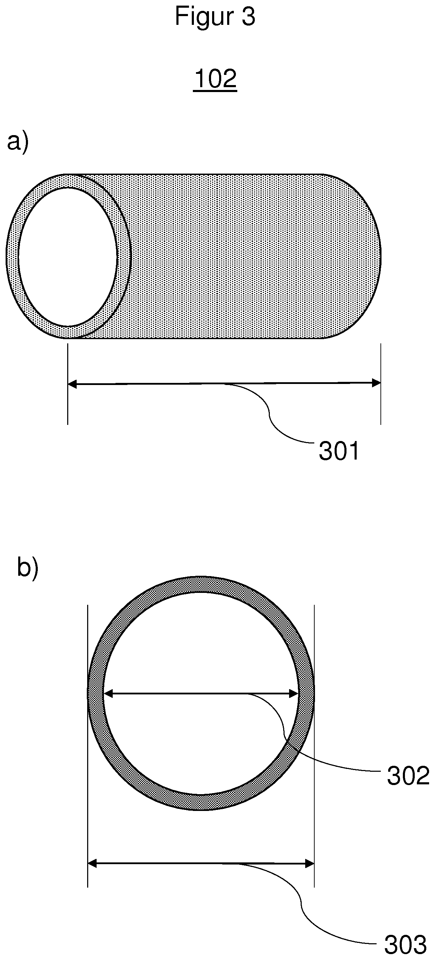

Figur 3 zeigt eine erfindungsgemäße Ablationselektrode, die im gezeigten Fall als Ringelektrode 102 ausgestaltet ist. Figur 3 a) zeigt eine perspektivische Ansicht der Ringelektrode 102. Figur 3 b) zeigt einen Querschnitt durch eine Ringelektrode 102. Generell können erfindungsgemäße Ablationselektroden 102, 103 wenigstens teilweise die Grundform eines Zylinders aufweisen, wobei die Elektroden wenigstens teilweise als Hohlzylinder ausgestaltet sind. Im gezeigten Fall ist die gesamte Elektrode als Hohlzylinder, auch Ringelektrode 102, ausgestaltet. Wie in Figur 3 a) gezeigt weist die Ringelektrode 102 eine Länge L 301 auf, die sich von einer Stirnseite des Hohlzylinders zur jeweils anderen Stirnseite erstreckt. Die Länge L beträgt in der Regel 3 bis 6 mm, andere Längen sind je nach Anwendungsfall möglich. Wie in Figur 3 b) gezeigt weist die Ringelektrode 102 einen kreisförmigen Querschnitt mit einem Außendurchmesser D 303 und einem Innendurchmesser d 302 auf. Die Differenz zwischen dem Außendurchmesser D 303 und dem Innendurchmesser d 302, geteilt durch zwei, definiert eine Wandstärke d_{W} der Ringelektrode 102. Erfindungsgemäße als Ringelektroden 102 ausgestaltete Ablationselektroden verfügen bevorzugt über Wandstärken dw von nicht mehr als 2 mm. Im gezeigten Fall beträgt die maximale Wandstärke d_{Wmax} gleich 0,3 mm. Die Wandung der Elektrode kann zusätzlich über durch Bohrung erzeugte Vertiefungen verfügen. In diesen Bereichen kann eine minimale Wandstärke d_{Wmin} von 80 µm erzeugt werden. Mit Ablationselektroden 102, 103 aus erfindungsgemäßen Goldlegierungen, insbesondere einer AuCo5 Legierung können auch über die gesamte Fläche der Wandung eine minimale Wandstärken d_{Wmin} von 80 µm aufweisen, das heißt derart geringe Wandstärken sind technisch realisierbar. Es versteht sich dass die erfindungsgemäßen Ablationselektroden 102, 103 nicht die Hohlzylinder- oder Ring-Form nach Figur 3 aufweisen müssen. Neben anderen sind halbkugelförmige Ausgestaltungen von Elektroden denkbar, wobei die Halbkugel in der Regel hohl ist und dadurch ähnlich einer Kugelschale ausgeformt ist. Derartige Elektroden sind insbesondere als Spitzen-Elektroden 103 in Ablations-Vorrichtungen 100 verwendbar, da diese einseitig geschlossen sind. Darüber hinaus sind Kombinationen der vorgenannten Formen möglich, wie beispielsweise als einseitig geschlossene Hohlzylinder ausgeformte Ablationselektroden 102, 103. Dabei kann die geschlossene Seite kantig oder abgerundet abschließen.

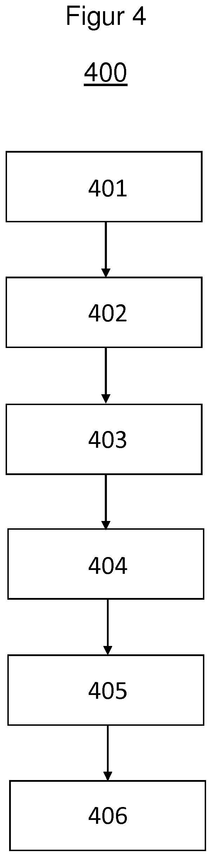

Figur 4 zeigt ein Ablaufdiagramm eines erfindungsgemäßen Verfahrens 400 zum Herstellen einer Ablationselektrode 102, 103, wobei die Ablationselektrode beispielsweise als Ringelektrode 102 ausgestaltet sein kann. In einem Verfahrensschritt a. 401 wird ein Vorläufer aus einer Goldlegierung bereitgestellt, wobei die Goldlegierung 95 Gew.-% Gold aufweist und 5 Gew.-% Kobalt aufweist. Der Vorläufer ist als Zylinder mit einem Querschnitts-Durchmesser von 6,0 bis 10,0 mm ausgestaltet ist und wird in einem Strangguss-Verfahren erhalten. In einem Verfahrensschritt b. 402 wird der Vorläufer zu einem drahtartigen Formkörper umgeformt, wobei ein Formkörper mit einem Querschnitts-Durchmesser von beispielsweise 2,0 bis 4,0 mm, je nach Anwendung, erhalten wird. In einem Verfahrensschritt c. 403 wird ein Lösungsglühen des Formkörpers durchgeführt. Das Lösungsglühen kann beispielsweise bei einer Temperatur von 950 °C für 60 min durchgeführt werden. In einem Verfahrensschritt d. 404 wird der lösungsgeglühte Formkörpers durch Eintauchen in ein flüssiges Medium um mehr als 500 °C abkühlt. Beispielsweise wird der lösungsgeglühte Formkörper ausgehend von 950 °C auf Zimmertemperatur durch Eintauchen in ein Wasserbad abgeschreckt. In einem Verfahrensschritt e. 405 wird der abgekühlte Formkörper bei einer Temperatur von 200 °C bis 600 °C für eine Dauer von wenigstens 0,5 h einer Auslagerungsglühung unterzogen um die Ausscheidungshärtung zu bewirken. Beispielsweise wird der 95 Gew.% Gold und 5 Gew.% Kobalt aufweisende Formkörper für 24 h bei einer Temperatur von 400 °C geglüht. In einem abschließenden Verfahrensschritt f. 406 wird der so erhaltene Formkörper zu einer Ablationselektrode geeigneter Geometrie, beispielsweise einer Ringelektrode 102, umgeformt.

### LISTE DER BEZUGSZEICHEN

- **100**: Katheter-Vorrichtung
- **101**: Katheter-Spitze
- **102**: Ringelektrode
- **103**: Spitzen-Elektrode
- **104**: Katheter-Körper
- **105**: Daumenauflage
- **106**: beweglicher Kolben
- **107**: Steuergriff
- **108**: Ausnehmung
- **109**: Elektrische Leitung
- **110**: Wandung
- **111**: Hohlraum
- **301**: Länge L
- **302**: Innendurchmesser d
- **303**: Außendurchmesser D
- **401**: Verfahrensschritt a.
- **402**: Verfahrensschritt b.
- **403**: Verfahrensschritt c.
- **404**: Verfahrensschritt d.
- **405**: Verfahrensschritt e.
- **406**: Verfahrensschritt f.

## Patentansprüche

1. Eine Ablationselektrode (102, 103) für die Hochfrequenzablation in der Medizintechnik, wobei die Ablationselektrode (102, 103) eine Goldlegierung umfasst,
**dadurch gekennzeichnet, dass**
die Goldlegierung ausscheidungsgehärtet ist, wenigstens 80 Gew.-% Gold aufweist und 1 Gew.-% bis 9 Gew.-% Kobalt aufweist.

2. Die Ablationselektrode (102, 103) nach Anspruch 1, **dadurch gekennzeichnet, dass** die Goldlegierung mindestens 90 Gew.-% Gold aufweist.

3. Die Ablationselektrode (102, 103) nach einem der Ansprüche 1 oder 2, **dadurch gekennzeichnet, dass** die Goldlegierung 2 Gew.% bis 8 Gew.% Kobalt aufweist, bevorzugt 4 Gew.% bis 6 Gew.% Kobalt aufweist, besonders bevorzugt 5 Gew.% Kobalt aufweist.

4. Die Ablationselektrode (102, 103) nach einem der Ansprüche 1 bis 3, **dadurch gekennzeichnet, dass** die Goldlegierung eine spezifische Wärmeleitfähigkeit von wenigstens 100 W/m*K aufweist, bevorzugt wenigstens 130 W/m*K aufweist, besonders bevorzugt wenigstens 150 W/m*K aufweist.

5. Die Ablationselektrode (102, 103) nach einem der Ansprüche 1 bis 4, **dadurch gekennzeichnet, dass** die Goldlegierung eine 0,2 %-Dehngrenze Rp_{0.2} von wenigstens Rp_{0.2} = 200 MPa aufweist, bevorzugt von wenigstens Rp_{0.2} = 500 MPa aufweist.

6. Die Ablationselektrode (102, 103) nach einem der Ansprüche 1 bis 5, **dadurch gekennzeichnet, dass** die Goldlegierung eine Zugfestigkeit Rₘ von wenigstens Rₘ = 400 MPa aufweist, bevorzugt von wenigstens Rₘ = 600 MPa aufweist.

7. Die Ablationselektrode (102, 103) nach einem der Ansprüche 1 bis 6, **dadurch gekennzeichnet, dass** die Elemente Gold und Kobalt in der Goldlegierung oberhalb von 800 °C einen einphasigen Mischkristall bilden und die Legierung bei Zimmertemperatur und/oder Körpertemperatur eine Ausscheidung metastabiler Phasen aufweist.

8. Die Ablationselektrode (102, 103) nach einem der Ansprüche 1 bis 7, **dadurch gekennzeichnet, dass** die Ablationselektrode (102, 103) wenigstens teilweise die Grundform eines Zylinders aufweist, wobei der Zylinder wenigstens über einen Teil seiner Länge als Hohlzylinder ausgebildet ist, wobei der Hohlzylinder einen Außendurchmesser D (303) und einen Innendurchmesser d (302) aufweist, wobei (D-d)/2 eine Wandstärke dw definieren, wobei eine maximale Wandstärke d_{Wmax} nicht mehr als d_{Wmax} = 2 mm beträgt, bevorzugt nicht mehr als d_{Wmax} = 1 mm beträgt, besonders bevorzugt nicht mehr als d_{Wmax} = 0,5 mm beträgt.

9. Eine Katheter-Spitze (101) zur Verwendung mit einer Katheter-Vorrichtung (100), umfassend eine Ablationselektrode (102, 103) nach einem der vorangehenden Ansprüche.

10. Die Katheter-Spitze (101) nach Anspruch 9, **dadurch gekennzeichnet, dass** die Katheter-Spitze (101) kein weiteres Kühlelement außer der Ablationselektrode (102, 103) aufweist.

11. Eine Katheter-Vorrichtung (100) für die Hochfrequenzablation, umfassend eine Katheter-Spitze (101) nach einem der Ansprüche 9 oder 10.

12. Ein Verfahren (400) zur Herstellung einer Ablationselektrode, wobei das Verfahren folgende Schritte (401, 402, 403, 404, 405, 406) beinhaltet:
a. Bereitstellen eines Vorläufers aus einer Goldlegierung, wobei die Goldlegierung wenigstens 80 Gew.-% Gold aufweist und 1 Gew.-% bis 9 Gew.-% Kobalt aufweist.
b. Formen des Vorläufers zu einem drahtförmigen Formkörper;
c. Lösungsglühen des Formkörpers bei einer Temperatur von 800 bis 1000 °C für wenigstens 15 min.
d. Abschrecken des lösungsgeglühten Formkörpers durch Eintauchen in ein flüssiges Medium, so dass der Formkörper um mehr als 500 °C abkühlt.
e. Auslagerungsglühen des abgeschreckten Formkörpers bei einer Temperatur von 200 °C bis 600 °C für eine Dauer von wenigstens 0,5 h.
f. Formen des auslagerungsgeglühten Formkörpers zu der Ablationselektrode (102, 103).

13. Das Verfahren (300) nach Anspruch 12, **dadurch gekennzeichnet, dass** das Auslagerungsglühen gemäß Schritt e. für eine Dauer von 15 h bis 30 h durchgeführt wird, bevorzugt für eine Dauer von 17 h bis 30 h durchgeführt wird, besonders bevorzugt für eine Dauer von 24 h bis 30 h durchgeführt wird.

14. Das Verfahren (300) nach einem der Ansprüche 12 oder 13 zur Herstellung der Ablationselektrode nach einem der Ansprüche 1 bis 8.

## Claims

1. An ablation electrode (102, 103) for high-frequency ablation in medical technology, whereby the ablation electrode (102, 103) comprises a gold alloy, **characterised in that**
the gold alloy is precipitation-hardened, comprises at least 80% by weight gold and 1% by weight to 9% by weight cobalt.

2. The ablation electrode (102, 103) according to claim 1, **characterised in that** the gold alloy comprises at least 90% by weight gold.

3. The ablation electrode (102, 103) according to either one of the claims 1 or 2, **characterised in that** the gold alloy comprises 2% by weight to 8% by weight cobalt, preferably comprises 4% by weight to 6% by weight cobalt, particularly preferably comprises 5% by weight cobalt.

4. The ablation electrode (102, 103) according to anyone of the claims 1 to 3, **characterised in that** the gold alloy comprises a specific thermal conductivity of at least 100 W/m*K, preferably at least 130 W/m*K, particularly preferably 150 W/m*K.

5. The ablation electrode (102, 103) according to anyone of the claims 1 to 4, **characterised in that** the gold alloy comprises a 0.2% proof stress R_{p0.2} of at least R_{p0.2} = 200 MPa, preferably of at least R_{p0.2} = 500 MPa.

6. The ablation electrode (102, 103) according to any one of the claims 1 to 5, **characterised in that** the gold alloy comprises a tensile strength Rₘ of at least Rₘ = 400 MPa, preferably of at least Rₘ = 600 MPa.

7. The ablation electrode (102, 103) according to anyone of the claims 1 to 6, **characterised in that** the elements gold and cobalt in the gold alloy form a single-phase mixed crystal above 800°C and **in that** the alloy comprises a precipitation of meta-stable phases at room temperature and/or body temperature.

8. The ablation electrode (102, 103) according to anyone of the claims 1 to 7, **characterised in that** the ablation electrode (102, 103) comprises, at least in part, the basic shape of a cylinder, whereby the cylinder is shaped as a hollow cylinder over a part of its length, whereby the hollow cylinder comprises an external diameter D (303) and an internal diameter d (302), whereby (D-d)/2 defines a wall thickness d_{w}, whereby a maximum wall thickness d_{wmax} is no more than d_{wmax} = 2mm, preferably no more than d_{wmax} = 1 mm, particularly preferably no more than d_{wmax} = 0.5 mm.

9. A catheter tip (101) for use with a catheter device (100), comprising an ablation electrode (102, 103) according to any one of the preceding claims.

10. The catheter tip (101) according to claim 9, **characterised in that** the catheter tip (101) comprises no further cooling element other than the ablation electrode (102, 103).

11. A catheter device (100) for high-frequency ablation, comprising a catheter tip (101) according to any one of the claims 9 or 10.

12. A method (400) for production of an ablation electrode, whereby the method includes the following steps (401, 402, 403, 404, 405, 406):
a. Providing a precursor made of a gold alloy, whereby the gold alloy comprises at least 80% by weight gold and 1% by weight to 9% by weight cobalt.
b. Moulding the precursor into a wire-like moulded body;
c. Solution annealing of the moulded body at a temperature from 800 to 1000°C for at least 15 min.
d. Quenching the solution-annealed moulded body by immersion in a liquid medium such that the moulded body cools down by more than 500°C.
e. Precipitation annealing of the quenched moulded body at a temperature from 200°C to 600°C for a period of at least 0.5 h.
f. Moulding the precipitation-annealed moulded body into the ablation electrode (102, 103).

13. The method (400) according to claim 12, **characterised in that** the precipitation annealing according to step e. is performed for a period from 15 h to 30 h, preferably for a period from 17 h to 30 h, particularly preferably for a period from 24 h to 30 h.

14. The method (400) according to any one of the claims 12 or 13 for production of the ablation electrode according to any one of the claims 1 to 8.

## Revendications

1. Une électrode d'ablation (102, 103) pour l'ablation par radiofréquence dans les technologies médicales, l'électrode d'ablation (102, 103) comprenant un alliage d'or,
**caractérisée en ce que**
l'alliage d'or est durci par précipitation, présente au moins 80% en poids d'or et présente de 1% en poids à 9% en poids de cobalt.

2. L'électrode d'ablation (102, 103) conformément à la revendication n°1, **caractérisée en ce que** l'alliage d'or présente au moins 90% en poids d'or.

3. L'électrode d'ablation (102, 103) conformément à l'une des revendications n°1 ou n°2, **caractérisée en ce que** l'alliage d'or présente de 2% en poids à 8% en poids de cobalt, de préférence de 4% en poids à 6% en poids de cobalt, en particulier de préférence 5% en poids de cobalt.

4. L'électrode d'ablation (102, 103) conformément à l'une des revendications n°1 à n°3, **caractérisée en ce que** l'alliage d'or présente une conductivité thermique spécifique d'au moins 100 W/m*K, de préférence d'au moins 130 W/m*K, en particulier de préférence d'au moins 150 W/m*K.

5. L'électrode d'ablation (102, 103) conformément à l'une des revendications n°1 à n°4, **caractérisée en ce que** l'alliage d'or présente une limite d'élasticité 0,2% Rp_{0.2} d'au moins Rp_{0.2} = 200 MPa, de préférence d'au moins Rp_{0.2} = 500 MPa.

6. L'électrode d'ablation (102, 103) conformément à l'une des revendications n°1 à n°5, **caractérisée en ce que** l'alliage d'or présente une résistance à la traction Rₘ d'au moins Rₘ = 400 MPa, de préférence d'au moins Rₘ = 600 MPa.

7. L'électrode d'ablation (102, 103) conformément à l'une des revendications n°1 à n°6, **caractérisée en ce que** les éléments or et cobalt forment un cristal mixte monophasé dans l'alliage d'or au-dessus de 800°C et que l'alliage présente une précipitation des phases métastables à température ambiante et/ou température corporelle.

8. L'électrode d'ablation (102, 103) conformément à l'une des revendications n°1 à n°7, **caractérisée en ce que** l'électrode d'ablation (102, 103) présente au moins partiellement la forme de base d'un cylindre, le cylindre étant conçu comme cylindre creux au moins sur une partie de sa longueur, le cylindre creux présentant un diamètre extérieur D (303) et un diamètre intérieur d (302), (D-d)/2 définissant une épaisseur de paroi d_{w}, une épaisseur de paroi maximale d_{wmax} ne dépassant pas d_{wmax} = 2 mm, de préférence ne dépassant pas d_{wmax} = 1 mm, en particulier de préférence ne dépassant pas d_{wmax} = 0,5 mm.

9. Une pointe de cathéter (101) pour une utilisation avec un dispositif de cathéter (100), comprenant une électrode d'ablation (102, 103) conformément à l'une des revendications précédentes.

10. La pointe de cathéter (101) conformément à la revendication n°9, **caractérisée en ce que** la pointe de cathéter (101) ne présente pas d'autre élément refroidissant en dehors de l'électrode d'ablation (102, 103).

11. Un dispositif de cathéter (100) pour l'ablation par radiofréquence, comprenant une pointe de cathéter (101) conformément à l'une des revendications n°9 ou n°10.

12. Un procédé (400) pour la fabrication d'une électrode d'ablation, le procédé englobant les étapes suivantes (401, 402, 403, 404, 405, 406) :
a. Mise à disposition d'un précurseur composé d'un alliage d'or, l'alliage d'or présentant au moins 80% en poids d'or et de 1% en poids à 9% en poids de cobalt.
b. Moulage du précurseur en un corps moulé filiforme.
c. Traitement thermique par mise en solution du corps moulé à une température de 800 à 1000°C pendant au moins 15 minutes.
d. Trempe du corps moulé traité thermiquement par mise en solution par une immersion dans un milieu liquide de façon à ce que le corps moulé refroidisse de plus de 500°C.
e. Traitement thermique par précipitation du corps moulé trempé à une température de 200°C à 600°C pendant une durée d'au moins 0,5 heure.
f. Moulage du corps moulé traité thermiquement par précipitation en une électrode d'ablation (102, 103).

13. Le procédé (400) conformément à la revendication n°12, **caractérisé en ce que** le traitement thermique par précipitation est réalisé conformément à l'étape e. pendant une durée de 15 h à 30 h, de préférence pendant une durée de 17 h à 30 h, en particulier de préférence pendant une durée de 24 h à 30 h.

14. Le procédé (400) conformément à l'une des revendications n°12 ou n°13 pour la fabrication de l'électrode d'ablation conformément à l'une des revendications n°1 à n°8.
